# EUROPEAN PATENT APPLICATION

(11) **EP 3 626 229 A1**
(43) Date of publication of application: **25.03.2020**
(21) Application number: 18802541.5
(22) Date of filing: 16.05.2018
(51) Int. Cl.: A61K 8/92, A61K 8/06, A61K 8/81, A61Q 19/00

(54) **OIL-IN-WATER EMULSION COMPOSITION**

(30) Priority: 19.05.2017 JP 2017100140
(71) Applicant: Shiseido Co., Ltd., Tokyo 104-0061 (JP)
(72) Inventor: IBE, Ayako, Yokohama-shi Kanagawa 224-8558 (JP); TAKAHASHI, Shigeo, Yokohama-shi Kanagawa 224-8558 (JP)
(74) Representative: Ter Meer Steinmeister & Partner
(86) International application number: PCT/JP2018/018875
(87) International publication number: WO 2018/212223

(57) **Abstract**

A purpose of the present invention is to provide an oil-in-water emulsion composition that is stable even when using solid oils from which it was conventionally considered to be difficult to form large oil-based particles exceeding 50 µm, and also improving the uniformity of the particle sizes and the shapes of the oil-based particles. The present invention relates to an oil-in-water emulsion composition in which oil-based particles containing a solid oil, a liquid oil and a powder are dispersed in a water phase, wherein: the oil-based particles have an average particle size of 50 µm to 10 mm; and the powder is contained inside the oil-based particles.

## Description

### TECHNICAL FIELD

The present invention relates to an oil-in-water emulsion composition. More specifically, the present invention relates to an oil-in-water emulsion composition comprising oil-based particles of which sizes are large enough to be visible, wherein each oil-based particle encloses a powder, and the uniformities of the respective shapes and sizes of the oil-based particles are significantly improved.

### BACKGROUND ART

An oil-in-water emulsion composition in which large oil-based particles having an average particle size of 50µm to 10 mm are dispersed in a water phase is known. Such an oil-in-water emulsion composition disperses large oil-based particles large enough to be visible, so that such a composition is not only creative and attractive from an aesthetic view, but also can provide an unprecedented sensation when used, i.e., a watery and fresh texture when applied to skin and a moist texture over time. Additionally, given decomposable oil-soluble components are held in the large oil-based particles, the effect of suppressing the decomposition of such oil-soluble components can be provided.

For example, Patent Document 1 describes a capsule-containing composition in which an oil-based component containing an amphiphilic substance such as behenyl alcohol, which is a solid at ambient temperature, is dispersed in an aqueous solvent dispersing oil-based capsules having an average particle size of 100µm or more.

In addition to Patent Document 1, Patent Document 2 describes that when large oil-based particles, having an average particle size of 0.05 to 10 mm, are dispersed in an oil-in-water emulsion composition, the stability and the uniformity of such particles were improved by blending a dextrin fatty acid ester into the oil phase. Patent Document 3 describes that, by blending a carboxyvinyl polymer and a polyoxyethylene-based associative thickener into the water phase in an oil-in-water emulsion composition, as well as the above patents, stickiness and filminess thereof were eliminated and the stability thereof was improved.

It is confirmed that each oil-based particle (oil-based capsule) in Patent Documents 1 to 3 has a structure comprising an outer layer (shell) that is an amphiphilic substance such as behenyl alcohol or a wax such as candelilla wax as the main component, and an inner layer (core) including a liquid oil such as a silicone oil as the main component.

However, even when using solid oils similar to behenyl alcohol and candelilla wax, oil-based particles having uniform shapes and sizes may not be formed depending on the type of oil, or the dispersibility of the formed oil-based particles may be poor and an aggregation thereof may occur, and the stability of the oil-based particles may be affected by the polarity or the like of the liquid oil included in the inner layer. Additionally despite improvements as disclosed in Patent Document 2, it is still difficult to make the formed oil-based particles to have uniform shapes and sizes.

### RELATED ART

### PATENT DOCUMENTS

Patent Document 1: JP 4798899 B
Patent Document 2: JP 2005-36001 A
Patent Document 3: JP 2012-67024 A

### SUMMARY OF THE INVENTION

### PROBLEM TO BE SOLVED BY THE INVENTION

Thus, the problem addressed by the present invention is that of making it possible to prepare an oil-in-water emulsion composition that is stable even when using solid oils that were conventionally deemed forming large oil-based particles exceeding 50µm to be difficult, and in addition, to improve the uniformity of the sizes and shapes of the oil-based particles.

### MEANS FOR SOLVING THE PROBLEM

As a result of performing diligent investigations towards solving the above-mentioned problems, the present inventors discovered that the aforementioned problems can be solved by blending (incorporating) a powder inside the oil-based particles, thereby completing the present invention.

In other words, the present invention provides an oil-in-water emulsion composition in which oil-based particles comprising a solid oil, a liquid oil and a powder are being dispersed in a water phase, wherein the oil-based particles have an average particle size in between 50µm and 10 mm; and the powder is enclosed inside the oil-based particles. Additionally, the present invention provides a cosmetic comprising the aforementioned oil-in-water emulsion composition as a (cosmetic) base.

### EFFECTS OF THE INVENTION

According to the present invention, even when using a solid oil that is conventionally deemed using to be difficult, production of an oil-in-water emulsion composition comprising large oil-based particles having an average particle size of 50µm to 10 mm becomes feasible. Furthermore, the oil-based particles included in the composition according to the present invention have an excellent uniformity in the shapes and sizes thereof, a good dispersibility, and are stable and not susceptible to aggregation.

### MODES FOR CARRYING OUT THE INVENTION

The composition according to the present invention is an oil-in-water emulsion composition in which oil-based particles having an average particle size of 50µm to 10 mm is dispersed in a water phase.

The oil-based particles (also sometimes referred to as the "internal phase", the "oil phase" or the "dispersed phase") in the present invention contain a solid oil, a liquid oil and a powder.

In the present invention, "solid oil" refers to oils that are solid or semi-solid at ambient temperature (25 °C).

Although the solid oil that is used in the present invention is not particularly limited, a solid oil having a melting point of 55 °C or higher is preferably used. Specific examples of the solid oil in the present invention include those named below, but the solid oil is not limited thereto.

Solid paraffin, microcrystalline wax, ceresin, beeswax, Bareco wax, polyethylene wax, silicone wax, higher alcohols (for example, behenyl alcohol, stearyl alcohol, cetyl alcohol, etc.), batyl alcohol, carnauba wax, cera alba, candelilla wax, jojoba wax, lanolin, shellac wax, spermaceti, Japan wax, higher fatty acids (for example, myristic acid, palmitic acid, stearic acid, behenic acid, 12-hydroxystearic acid, etc.), ester oils (for example, myristyl myristate, etc.), cacao fat, hardened castor oil, hardened oils, hydrogenated palm oil, palm oil, hardened coconut oil, polyetheylene, vaseline, various hydrogenated animal or vegetable oils and fats, aliphatic monocarboxylic acid lanolin alcohol esters, and the like.

Among the above, solid oils having a melting point of 50 °C or higher are preferred, and solid oils having a melting point of 65 °C or higher and lower than 85 °C are particularly preferred. If the melting point is lower than 50 °C, there is a tendency for the stability to be poor at high temperatures. Conversely, if the melting point is higher than 85 °C, there is a tendency for problems to occur in the particle formation and the dispersibility. Although there are no limits on the preferred solid oils, examples include hydrogenated jojoba oil (melting point 68 °C), glyceryl behenate/eicosadioate (melting point 66 °C), higher alcohols having 16 or more carbon atoms, preferably 18 or more carbon atoms, such as stearyl alcohol (melting point 52 to 62 °C) and behenyl alcohol (melting point 68 °C), microcrystalline wax (melting point 80 °C), ceresin (melting point 68 to 75 °C), polyethylene wax (melting point 80 °C), batyl alcohol (melting point 70 °C), carnauba wax (melting point 83 °C), candelilla wax (melting point 71 °C), hardened castor oil (melting point 84 °C), stearic acid (melting point 58 to 63 °C), behenic acid (melting point 69 to 80 °C) and 12-hydroxystearic acid (melting point 70 °C).

These solid oils may be used as one type alone or as a combination of two or more types.

The blended amount of the solid oil in the composition of the present invention is preferably 5 to 50% by mass, more preferably 10 to 20% by mass relative to the overall mass of the oil phase (excluding the powder). If the blended amount of the solid oil is less than 5% by mass, there is a tendency for the stability of the composition to become worse, and if it exceeds 50% by mass, there is a tendency for the oil-based particles to become too hard, thereby making the texture and the skin compatibility worse.

In the present invention, "liquid oil" refers to oils that are liquid at ambient temperature (25 °C). Specific examples of the liquid oil used in the present invention include those named below, but the liquid oil is not limited thereto.

Oils and fats such as linseed oil, camellia oil, macadamia nut oil, corn oil, olive oil, avocado oil, sasanqua oil, castor oil, safflower oil, apricot kernel oil, cinnamon oil, jojoba oil, grape oil, almond oil, rapeseed oil, sesame oil, sunflower oil, wheat germ oil, rice germ oil, rice bran oil, cottonseed oil, soybean oil, peanut oil, tea seed oil, evening primrose oil, triglycerin, glyceryl trioctanoate, glyceryl triisopalmitate, coconut oil, palm oil, palm kernel oil.

Ester oils such as octanoic acid esters such as cetyl octanoate, isooctanoic acid esters such as glyceryl tri-2-ethylhexanoate and pentaerythrityl tetraethylhexanoate, lauric acid esters such as hexyl laurate, myristic acid esters such as isopropyl myristate and octyldodecyl myristate, palmitic acid esters such as octyl palmitate, stearic acid esters such as isocetyl stearate, isostearic acid esters such as isopropyl isostearate, isopalmitic acid esters such as octyl isopalmitate, oleic acid esters such as isodecyl oleate, adipic acid diesters such as diisopropyl adipate, sebacic acid diesters such as diethyl sebacate, and malic acid diesters such as diisostearyl malate.

Hydrocarbon oils such as liquid paraffin, ozokerite, squalane, squalene, pristane, paraffin, isoparaffin and vaseline.

Silicone oils such as chain silicones such as dimethylpolysiloxane (dimethicone), methyl phenyl polysiloxane (phenyl methicone) and methyl hydrogen polysiloxane, cyclic silicones such as decamethylcyclopentasiloxane and dodecamethylcyclohexasiloxane, and modified silicones such as amino-modified silicone oils, polyether-modified silicone oils, carboxy-modified silicone oils, alkyl-modified silicone oils, ammonium salt-modified silicone oils and fluorine-modified silicone oils.

These liquid oils may be used alone as a single kind of oil or as a combination of two or more kinds of oils.

The blended amount of the liquid oil is preferably 50 to 95% by mass, more preferably 60 to 90% by mass relative to the overall mass of the oil phase (excluding the powder). If the blended amount of the liquid oil is less than 50% by mass, there is a tendency for the oil-based particles to become too hard, thereby making the texture and the skin compatibility worse, and if it exceeds 95% by mass, there is a tendency for the stability of the composition to become worse.

In the present invention, the "powder" is not particularly limited as long as it can be blended into an external preparation for use on skin, such as a cosmetic.

Examples include inorganic powders such as talc, mica, kaolin, mica, sericite, white mica, phlogopite, synthetic mica, lepidolite, biotite, lithia mica, vermiculite, magnesium carbonate, calcium carbonate, aluminum silicate, barium silicate, calcium silicate, magnesium silicate, strontium silicate, tungstic acid metal salts, magnesium, spherical silica, zeolite, barium sulfate, calcined calcium sulfate (calcined plaster), calcium phosphate, fluorapatite, hydroxyapatite, ceramic powder, metal soap (such as zinc myristate, calcium palmitate or aluminum stearate) and boron nitride; organic spherical powders such as spherical polyamide resin powder (spherical nylon powder), spherical polyethylene, spherical crosslinked poly methyl (meth)acrylate resin powder, spherical polyester, spherical crosslinked polystyrene resin powder, spherical styrene-acrylic acid copolymer resin powder, spherical benzoguanamine resin powder, spherical polytetrafluoroethylene powder and spherical cellulose; inorganic white pigments such as titanium dioxide and zinc oxide; inorganic red pigments such as iron oxide (red iron oxide) and iron titanate; inorganic brown pigments such as γ-iron oxide; inorganic yellow pigments such as yellow iron oxide and ocher; inorganic black pigments such as black iron oxide, carbon black and lower titanium oxides; inorganic violet pigments such as mango violet and cobalt violet; inorganic green pigments such as chromium oxide, chromium hydroxide and cobalt titanate; inorganic blue pigments such as ultramarine blue and Prussian blue; pearlescent pigments such as titanium oxide-coated mica, titanium oxide-coated bismuth oxychloride, titanium oxide-coated talc, colored titanium oxide-coated mica, bismuth oxychloride, (Ca/Al) borosilicate and fish scale flakes; metal powder pigments such as aluminum powder and copper powder; red, yellow, orange, yellow, green or blue colorants, or colorants (organic pigments) in which these are formed into lakes with zirconium, barium, aluminum or the like; and natural pigments such as chlorophyll and β-carotene. Among these, talc is particularly preferred.

The powder in the present invention may or may not be surface-treated, and the shape thereof is not particularly limited. The average particle size of the powder is not particularly limited, but normally, a powder of approximately 1 to 100µm is preferably used.

The blended amount of the powder is preferably 0.12 to 30% by mass, more preferably 0.15 to 15% by mass, and even more preferably 0.3 to 9% by mass relative to the overall mass of the oil phase (excluding the powder). If the blended amount of the powder in the oil phase is less than 0.12% by mass, there is a tendency for the dispersibility of the oil-based particles to be reduced and for aggregation to easily occur. Conversely, if the blended amount exceeds 30% by mass, there is a tendency for the composition to be difficult to handle during preparation, and for the particle sizes and variability to become greater.

The water phase (also sometimes referred to as the "external phase" or the "continuous phase") in the composition of the present invention contains water and/or an aqueous medium as the main component, and serves as the dispersion medium for the oil-based particles.

The blended amount of the water constituting the water phase in the composition of the present invention is not particularly limited, but is normally 50 to 99% by mass, preferably 60 to 98% by mass, more preferably 70 to 95% by mass relative to the overall mass of the water phase.

The composition of the present invention may contain, in addition to the above-mentioned essential components, other components that can be blended into external preparations for use on skin, such as cosmetics, within a range not inhibiting the effects of the present invention.

Although the other components that can be blended into the composition of the present invention are not limited, examples include water-soluble thickeners, oil-soluble thickeners, humectants, water-soluble medicinal agents (such as, for example, arbutin, ascorbic acid glucoside, tranexamic acid and 4-methoxysalicylic acid salts), oil-soluble medicinal agents (such as, for example, oil-soluble vitamins and oil-soluble vegetable extracts), ultraviolet absorption agents, chelating agents such as sodium edetate, pH adjusters such as citric acid/sodium citrate, preservatives such as paraben and phenoxyethanol, colorants, dyes, fragrances, surfactants, and the like.

Among the above-mentioned other components, it is preferable to blend a water-soluble thickener and/or an oil-soluble thickener. A water-soluble thickener can raise the viscosity of the composition and further improve the stability of the dispersion.

Although the water-soluble thickener that can be blended in the present invention is not particularly limited, examples include vegetable polymers such as gum arabic, tragacanth gum, galactan, carob gum, guar gum, karaya gum, carrageenan, pectin, agar, quinceseed (marmelo) and algecolloid (brown algae extract); microbial polymers such as dextran, succinoglucan and pullulan; animal polymers such as collagen, casein, albumin and gelatin; cellulose polymers such as methylcellulose, nitrocellulose, ethylcellulose, methylhydroxypropylcellulose, hydroxyethylcellulose, sodium cellulose sulfate, hydroxypropylcellulose, sodium carboxymethylcellulose, crystalline cellulose and cellulose powder; alginate polymers such as sodium alginate and alginic acid propylene glycol esters; vinyl polymers such as polyvinyl alcohol, polyvinyl methyl ether, polyvinylpyrrolidone, carboxyvinyl polymers and alkyl-modified carboxyvinyl polymers; acrylic polymers such as polyoxyethylene polymers, polyoxyethylene-polyoxypropylene copolymers, sodium polyacrylate, poly ethyl acrylate and polyacrylamide; and inorganic water-soluble polymers such as polyethyleneimine, cationic polymers, bentonite, aluminum-magnesium silicate, laponite, hectorite and silicic anhydride.

Among water-soluble thickeners, an alkyl-modified carboxyvinyl polymer is active to suppress aggregation/coalescence of the oil-based particles based on the surface activity thereof, so that such a polymer is particularly preferred. Alkyl-modified carboxyvinyl polymers are also known as acrylate/alkyl methacrylate copolymers (acrylates/C10-30 alkyl acrylate crosspolymers), and are commercially available, for example, under the trade names Carbopol® 1342, Pemulen® TR-1 and Pemulen® TR-2 (manufactured by BF Goodrich®).

The amount of the water-soluble thickener can be appropriately adjusted in accordance with the type of water-soluble thickener, the purpose of use, and the desired texture, and there are no particular limitations thereto. Normally, the amount is 0.001 to 2% by mass, preferably 0.01 to 1.3% by mass relative to the overall mass of the composition.

The oil-soluble thickener can be adjusted to a suitable hardness by lowering the solidification power of the solid oil constituting the oil-based particles.

Although the oil-soluble thickener that can be blended in the present invention is not particularly limited, examples include dextrin fatty acid esters, metal soaps, lipophilic bentonites, amino acid derivatives, sucrose fatty acid esters and benzylidene derivatives of sorbitol.

Examples of dextrin fatty acid esters include dextrin palmitic acid esters, dextrin oleic acid esters and dextrin stearic acid esters.

Examples of metal soaps include aluminum stearate having residual hydroxyl groups, magnesium stearate, zinc myristate and the like. Examples of lipophilic bentonites include dimethylbenzyldodecylammonium montmorillonite, dimethyldioctadecylammonium montmorillonite, and the like.

Examples of amino acid derivatives include, for example, N-lauroyl-L-glutamic acid, α,γ-di-n-butylamine, and the like.

Examples of sucrose fatty acid esters include, for example, an ester of which not more than three out of eight hydroxy groups in sucrose are esterified with a higher fatty acid, e.g., stearic acid and palmitic acid.

Examples of benzylidene derivatives of sorbitol include monobenzylidene sorbitol, dibenzylidene sorbitol, and the like.

Among these, dextrin palmitate is particularly preferred in view of the stability and texture. The dextrin fatty acid esters are not particularly limited, but for example, those that are commercially available from Chiba Flour Milling Co., Ltd. under the tradenames "Rheopearl® KL", "Rheopearl® KE" or the like may be used.

The amount of the oil-soluble thickener can be appropriately adjusted in accordance with the purpose of use and the desired sensation when used, and there are no particular limitations thereon. Normally, the amount should be 0.01 to 5% by mass, preferably 0.02 to 2% by mass relative to the overall mass of the oil phase.

The composition of the present invention can be prepared, for example, in accordance with the methods described in Patent Documents 1 to 3. Specifically, it can be prepared by mixing the water phase component, heating the water phase up to a higher temperature than the melting point of the solid oil to be blended, adding the mixed oil phase component (including the solid oil, the liquid oil and the powder) heated to the same temperature as the water phase component under stirring (being implemented at a rotation rate 10 to 1500 rpm and preferably approximately 20 to 300 rpm using a propeller or paddle mixer, but a homomixer is inadequate due to that the particles are pulverized too much). Next, cooling the mixture under stirring enables a preparation.

The composition of the present invention that is produced in this manner is a dispersion of oil-based particles, having an average particle size of 50µm to 10 mm, in a water phase. The oil-based particles in the composition of the present invention is deemed having a capsule structure in which an inner layer comprises substantially the powder and the liquid oil, and the periphery thereof (outer layer) is coated with a solid oil. Additionally, the solid oil forming the outer layer crystallizes, so that a hardness of the oil-based particle becomes suitable so as to provide a unique texture when used.

With regard to the composition of the present invention, oil-based particles having the abovementioned structure are formed even without using a surfactant and in addition, the powder is present inside (in the inner layer of) each oil-based particle and a solid oil is present at the interface between the water phase and the oil phase, so that the present composition is clearly distinguishable from a Pickering emulsion, in which a powder is present at the oil-water interface.

In the present invention, if a colorant such as a pigment is blended as the powder, it is preferable to use a small amount (for example, 0.005% by mass or less) of a surfactant. Such a surfactant is preferably the lipophilic surfactant having an HLB of 7 or lower, and specific examples include PEG-9 polydimethylsiloxyethyl dimethicone, sorbitan sesquiisostearate, and the like.

The oil-in-water emulsion composition of the present invention is appropriate for use as a cosmetic base having a unique appearance and sensation when used. Examples of cosmetics in which said base is used include aqueous cosmetics, gel-type cosmetics and emulsion cosmetics. Specific uses include facial, body or hair cosmetics such as lotions, gels, milky lotions, creams and packs.

### EXAMPLES

While the present invention will be explained in further detail by providing examples below, the present invention is not limited by these examples in any way. Where not otherwise noted, the blended amounts are indicated in % by mass relative to the overall mass of the composition in which those components are blended.

Oil-in-water emulsion compositions were prepared in accordance with the method described in paragraph [0042], using the formulations indicated in Table 1 to Table 3 below. The emulsion composition of each example was evaluated in the below-indicated categories (1) and (2) according to the below-indicated criteria.

### (1) Oil-based particle formation properties

The particle sizes and shapes of the oil-based particles contained in the prepared emulsion compositions were observed under a microscope and the average particle sizes were computed.

### Evaluation criteria:

A+: Particles were satisfactorily formed, the average particle size of the formed particles was in between 50µm and 10 mm, and the particles having the size of the average particle size ±10% are at least 90%.
A: Particles were satisfactorily formed, the average particle size of the formed particles was in between 50µm and to 10 mm, and the particle having the size of the average particle size ± 10% was at least 80% and less than 90%.
B: Particles were satisfactorily formed, the average particle size of the formed particles was in between 50µm and 10 mm, and the particle having the size of the average particle size ± 10% was at least 50% and less than 80%.
C: The oil phase forming a particle was 10% to 50% and the rest thereof formed a lump.
D: Almost all oil phases formed a lump, and less than 10% of the oil phases formed a particle.

### (2) Dispersibility

After letting the prepared emulsion composition stand at ambient temperature for one week, the dispersibility when the composition was agitated by shaking the container with the hand was visually observed.
A: The particles immediately evenly dispersed.
B: Some of the particles were aggregated, but became evenly dispersed when more vigorously agitated.
C: Some of the particles were aggregated and did not disperse even when more vigorously agitated.
D: The particles were completely aggregated and did not disperse at all, even when more vigorously agitated.

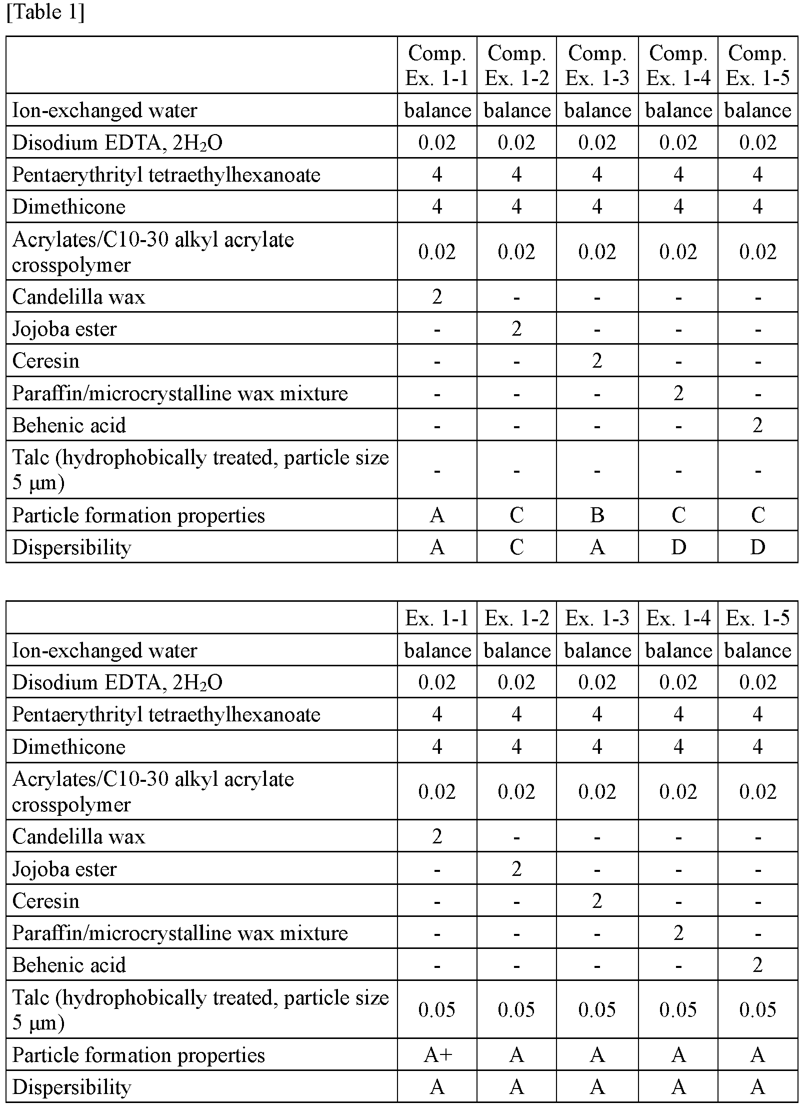

As indicated in Table 1, compared to Comparative Example 1-1 using candelilla wax, which has conventionally been known to form oil-based particles, Example 1-1, in which a powder was further blended, had improved particle uniformity and the particle shapes were all spherical. Meanwhile, even when using a solid oil (Comparative Examples 1-2 to 1-5), which has conventionally been thought to be difficult to form into oil-based particles, satisfactory oil-based particles were formed by blending in a powder (Examples 1-2 to 1-5).

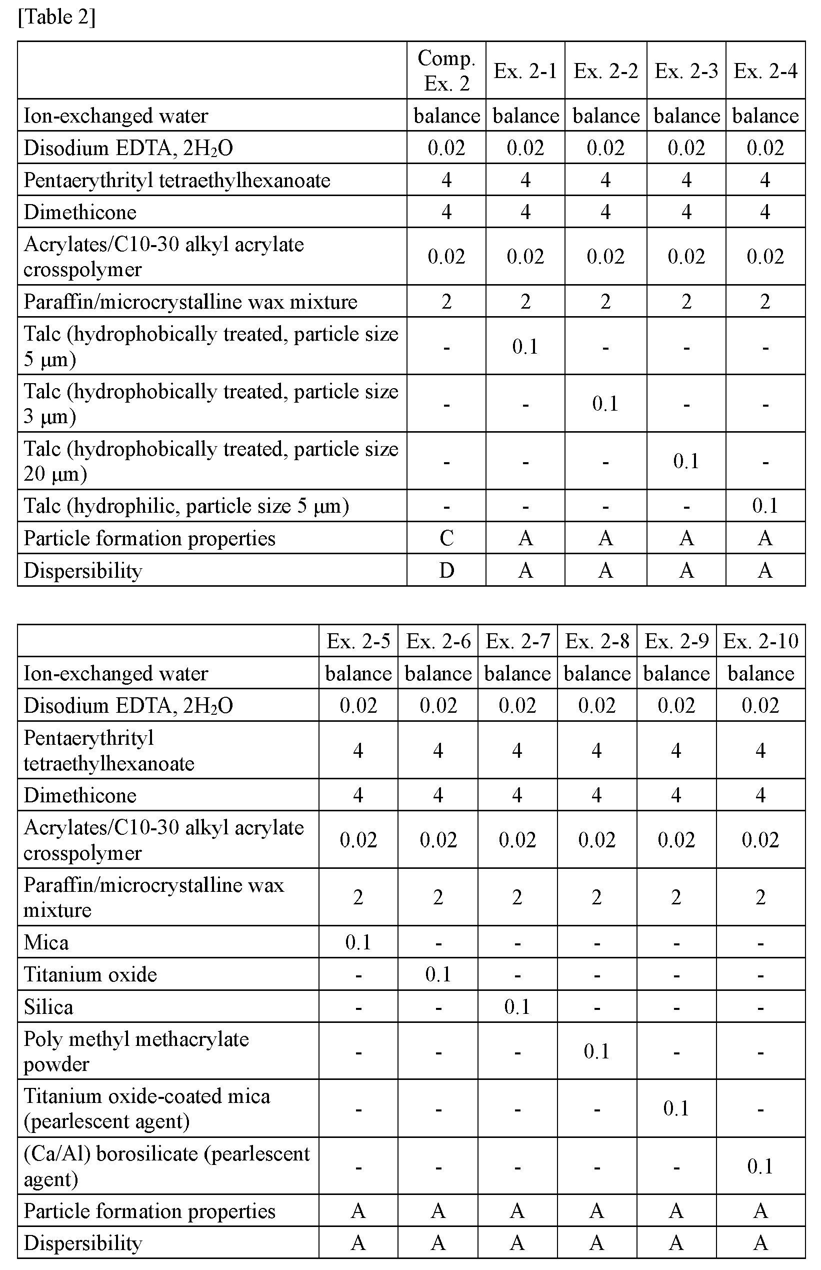

As shown in Table 2, even when the types (material or particle sizes) of the blended powders were changed, satisfactory oil-based particles were able to be formed and excellent dispersibility was also obtained. However, oil-based particles were not able to be formed in Comparative Example 2, in which a powder was not blended.

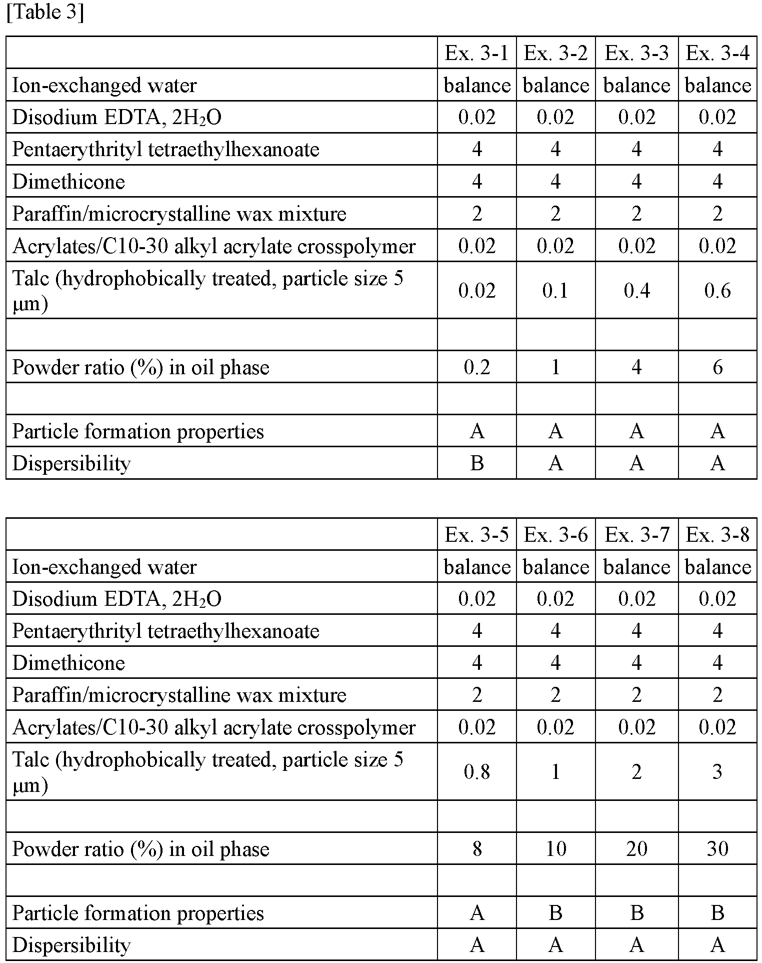

As shown in Table 3, even when the amount of the blended powder (the blending ratio of the powder relative to the oil phase (excluding the powder)) was varied over a certain range, there was no problem in the formation properties and the dispersibility of the oil-based particles.

Next, oil-in-water emulsion compositions were prepared with the formulations indicated in Table 4 and Table 5 below, and the (1) oil-based particle formation properties and the (2) dispersibility were evaluated in accordance with the same criteria as those indicated above. In addition, the (3) stability of the emulsion compositions was evaluated by the criteria indicated below.

### (3) Stability

After storing the emulsion composition of each example for one month at 25 °C and 40 °C, the state of the oil-based particles was visually compared with the state immediately after preparation, and the stability was evaluated according to the criteria indicated below.
A: No change was observed in the particles under any storage condition.
B: Less than 20% of the particles was observed to have aggregation/deformation only when stored at 40 °C.
C: At least 20% of the particles was observed to have aggregation/deformation only when stored at 40 °C.
D: At least 20% of the particles was observed to have aggregation/deformation under any storage condition.

**[Table 4]**

| | Ex. 4-1 | Ex. 4-2 | Ex. 4-3 | Ex. 4-4 |
|---|---|---|---|---|
| Ion-exchanged water | balance | balance | balance | balance |
| Disodium EDTA, 2H₂O | 0.02 | 0.02 | 0.02 | 0.02 |
| Pentaerythrityl tetraethylhexanoate | 4 | 4 | 4 | 4 |
| Dimethicone | 4 | 4 | 4 | 4 |
| Acrylates/C10-30 alkyl acrylate crosspolymer | 0.02 | 0.02 | 0.02 | 0.02 |
| Myristyl myristate (melting point 43 °C) | 2 | - | - | - |
| Stearic acid (melting point 58 to 63 °C) | - | 2 | - | - |
| Cetanol (melting point 48 to 54 °C) | - | - | 2 | - |
| Behenyl alcohol (melting point 65 to 73 °C) | - | - | - | 2 |
| Talc (hydrophobically treated, particle size 5 µm) | 0.1 | 0.1 | 0.1 | 0.1 |
| Particle formation properties | A | A | B | A |
| Dispersibility | A | A | A | A |
| Stability | C | A | B | A |

As shown in Table 4, oil-based particles were satisfactorily formed and the dispersibility was also satisfactory in all of Examples 4-1 to 4-4. However, Example 4-1, which used a solid oil with a melting point lower than 50 °C, had inferior stability at a high temperature.

**[Table 5]**

| | Comp. Ex. 5-1 | Comp. Ex. 5-2 | Ex. 5-1 | Ex. 5-2 |
|---|---|---|---|---|
| Ion-exchanged water | balance | balance | balance | balance |
| Disodium EDTA, 2H₂O | 0.02 | 0.02 | 0.02 | 0.02 |
| Pentaerythrityl tetraethylhexanoate | 4 | 4 | 4 | 4 |
| Dimethicone | 4 | 4 | 4 | 4 |
| Acrylates/C10-30 alkyl acrylate crosspolymer | 0.02 | 0.02 | 0.02 | 0.02 |
| Ceresin | 2 | 2 | 2 | 2 |
| Fragrance | - | 1 | 1 | 1 |
| Talc (hydrophobically treated, particle size 5 | - | - | 0.1 | - |
| Mica (hydrophobically treated, particle size 5 µm) | - | - | - | 0.1 |
| Particle formation properties | B | C | A | A |
| Dispersibility | A | D | A | A |
| Stability | B | - | A | A |

Comparative Example 5-1, in which ceresin was used as the solid oil, was able to form oil-based particles even without blending in a powder, but oil-based particles were not satisfactorily formed when a high-polarity fragrance was added to the oil phase (Comparative Example 5-2). However, by blending in a powder, oil-based particles were further satisfactorily formed, even when a fragrance was blended, and the dispersibility and the stability were also excellent.

Hereinbelow, formulations of cosmetics using the emulsion composition of the present invention will be described, but the present invention is not limited to these examples.

**Formulation Example 1: Essence**

| Water phase | | |
|---|---|---|
| 1. | Ion-exchanged water | balance |
| 2. | Glycerin | 5 |
| 3. | Propylene glycol | 5 |
| 4. | Carboxyvinyl polymer | 0.2 |
| 5. | Xanthan gum | 0.2 |
| 6. | Hydroxyethylcellulose | 0.2 |
| 7. | Inositol | 0.1 |
| 8. | Disodium EDTA, 2H₂O | 0.05 |
| 9. | Sodium hydroxide | 0.1 |
| 10. | Methyl paraben | 0.2 |

| Oil phase | | |
|---|---|---|
| 11. | Pentaerythrityl tetraethylhexanoate | 3 |
| 12. | Phenyl methicone | 2 |
| 13. | Liquid paraffin | 1 |
| 14. | Microcrystalline wax | 2 |
| 15. | Acrylates/C10-30 alkyl acrylate crosspolymer | 0.1 |
| 16. | Mica | 0.5 |
| 17. | Retinol | 0.2 |

**Formulation Example 2: Whitening cosmetic gel**

| Water phase | | |
|---|---|---|
| 1. | Ion-exchanged water | balance |
| 2. | Alcohol | 5 |
| 3. | Glycerin | 10 |
| 4. | 1,3-butylene glycol | 5 |
| 5. | Tranexamic acid | 1 |
| 6. | Dimethylacrylamide/sodium acryloyldimethyl taurate crosspolymer | 0.5 |
| 7. | Disodium EDTA, 2H2O | 0.05 |
| 8. | Methyl paraben | 0.2 |

| Oil phase | | |
|---|---|---|
| 9. | Isopropyl myristate | 3 |
| 10. | Macadamia nut oil | 1 |
| 11. | Decamethylcyclopentasiloxane | 1 |
| 12. | Squalane | 0.5 |
| 13. | Behenic acid | 1.5 |
| 14. | Acrylates/C10-30 alkyl acrylate crosspolymer | 0.1 |
| 15. | Mica | 0.2 |
| 16. | Dextrin palmitic acid ester | 0.01 |

**Formulation Example 3: Essence**

| Water phase | | |
|---|---|---|
| 1. | Ion-exchanged water | balance |
| 2. | Glycerin | 5 |
| 3. | Propylene glycol | 5 |
| 4. | Carboxyvinyl polymer | 0.2 |
| 5. | Xanthan gum | 0.2 |
| 6. | Hydroxyethylcellulose | 0.2 |
| 7. | Inositol | 0.1 |
| 8. | Disodium EDTA, 2H₂O | 0.05 |
| 9. | Sodium hydroxide | 0.1 |
| 10. | Methyl paraben | 0.2 |

| Oil phase | | |
|---|---|---|
| 11. | Pentaerythrityl tetraethylhexanoate | 3 |
| 12. | Phenyl methicone | 2 |
| 13. | Liquid paraffin | 1 |
| 14. | Microcrystalline wax | 2 |
| 15. | Acrylates/C10-30 alkyl acrylate crosspolymer | 0.1 |
| 16. | Mica | 0.5 |
| 17. | Retinol | 0.2 |

**Formulation Example 4: Whitening cosmetic gel**

| | | |
|---|---|---|
| 1. | Ion-exchanged water | balance |
| 2. | Alcohol | 5 |
| 3. | Glycerin | 10 |
| 4. | 1,3-butylene glycol | 5 |
| 5. | Tranexamic acid | 1 |
| 6. | Dimethylacrylamide/sodium acryloyldimethyl taurate crosspolymer | 0.5 |
| 7. | Disodium EDTA, 2H2O | 0.05 |
| 8. | Methyl paraben | 0.2 |

| Oil phase | | |
|---|---|---|
| 9. | Isopropyl myristate | 3 |
| 10. | Macadamia nut oil | 1 |
| 11. | Decamethylcyclopentasiloxane | 1 |
| 12. | Squalane | 0.5 |
| 13. | Behenic acid | 1.5 |
| 14. | Acrylates/C10-30 alkyl acrylate crosspolymer | 0.1 |
| 15. | Mica | 0.2 |
| 16. | Dextrin palmitic acid ester | 0.01 |

**Formulation Example 5: Whitening essence**

| Water phase | | |
|---|---|---|
| 1. | Ion-exchanged water | balance |
| 2. | Glycerin | 2 |
| 3. | Dipropylene glycol | 8 |
| 4. | Agar | 0.4 |
| 5. | Sodium poly-γ-glutamate | 0.1 |
| 6. | Allantoin | 0.1 |
| 7. | Sodium 4-methoxysalicylate | 1 |
| 8. | Sodium metaphosphate | 0.2 |
| 9. | Phenoxyethanol | 0.3 |
| Oil phase | | |
| 10. | Cetyl ethylhexanoate | 2 |
| 11. | Dimethyl polysiloxane | 1 |
| 12. | Isohexadecane | 1 |
| 13. | (Dimethicone/vinyl dimethicone) crosspolymer | 0.5 |
| 14. | Beeswax | 2 |
| 15. | Acrylates/C10-30 alkyl acrylate crosspolymer | 0.2 |
| 16. | Titanium oxide | 0.5 |
| 17. | Tocopherol | 0.01 |
| 18. | Fragrance | 0.1 |

**Formulation Example 6: Cosmetic gel**

| Water phase | | |
|---|---|---|
| 1. | Ion-exchanged water | balance |
| 2. | Diglycerin | 1 |
| 3. | Dipropylene gycol | 5 |
| 4. | Tetramethyl pyrazolyl pyrimidine HCL | 0.6 |
| 5. | (Sodium acrylate/sodium acryloyldimethyl taurate) copolymer | 1.5 |
| 6. | Disodium EDTA, 2H2O | 0.1 |
| 7. | Methyl paraben | 0.2 |

| Oil phase | | |
|---|---|---|
| 8. | Triethylhexanoin | 1 |
| 9. | Diisostearyl malate | 0.5 |
| 10. | Olive oil | 0.05 |
| 11. | Squalane | 2 |
| 12. | Methyl phenyl polysiloxane | 2 |
| 13. | Trimethylsiloxy silicic acid | 0.2 |
| 14. | Polyethylene wax | 1.2 |
| 15. | Acrylates/C10-30 alkyl acrylate crosspolymer | 0.1 |
| 16. | Poly methyl methacrylate | 0.4 |

**Formulation Example 7: Makeup base**

| Water phase | | |
|---|---|---|
| 1. | Ion-exchanged water | balance |
| 2. | Alcohol | 4 |
| 3. | Glycerin | 2 |
| 4. | Dipropylene glycol | 5 |
| 5. | Marine collagen | 1 |
| 6. | Sodium acrylate graft starch | 0.5 |
| 7. | Dimethylacrylamide/sodium acryloyldimethyl taurate crosspolymer | 0.5 |
| 8. | Disodium EDTA, 2H₂O | 0.2 |
| 9. | Methyl paraben | 0.2 |

| Oil phase | | |
|---|---|---|
| 10. | Cetyl ethylhexanoate | 1 |
| 11. | Glyceryl diisostearate | 0.5 |
| 12. | Liquid paraffin | 2 |
| 13. | Dimethicone | 2 |
| 14. | 2-Ethylhexyl para-methoxycinnamate | 5 |
| 15. | Carnauba wax | 1.5 |
| 16. | Acrylates/C10-30 alkyl acrylate crosspolymer | 0.05 |
| 17. | PEG-9 polydimethylsiloxyethyl dimethicone (lipophilic surfactant) | 0.003 |
| 18. | Titanium oxide | 0.4 |
| 19. | Iron oxide (pigment) | 0.01 |
| 20. | Fragrance | 0.1 |

## Claims

1. An oil-in-water emulsion composition in which oil-based particles containing a solid oil, a liquid oil and a powder are dispersed in a water phase, wherein:
the oil-based particles have an average particle size of 50 µm to 10 mm; and
the powder is contained inside the oil-based particles.

2. The composition according to claim 1, wherein the solid oil is selected from among solid oils having a melting point of 50 °C or higher.

3. The composition according to claim 1 or 2, wherein the blended amount of the powder is 0.12 to 30% by mass relative to the overall mass of the oil phase.

4. The composition according to any one of claims 1 to 3, further containing an alkyl-modified carboxyvinyl polymer.

5. The composition according to any one of claims 1 to 4, further containing 0.005% by mass or less of a surfactant.

6. The composition according to claim 5, wherein the surfactant is a surfactant having an HLB of 7 or lower.

7. A cosmetic containing the composition according to any one of claims 1 to 6 as a base.

8. The cosmetic according to claim 7, which is an aqueous cosmetic, a gel cosmetic or an emulsion cosmetic.
